# EUROPÄISCHE PATENTSCHRIFT

(11) **EP 0 539 803 B1**
(45) Veröffentlichungstag und Bekanntmachung des Hinweises auf die Patenterteilung: **07.01.1999**
(21) Anmeldenummer: 92117606.1
(22) Anmeldetag: 15.10.1992
(51) Int. Cl.: C07D 471/10, A61K 31/435

(54) **Triazaspirodecanon-Methyl-Chromane zur Bekämpfung von Erkrankungen des Zentralnervensystems**
Triazospirodecanone-methyl-chromans for the treatment of central nervous system illnesses
Triazaspirodecanone-méthyl-chromanes pour le traitement des maladies du système nerveux central

(30) Priorität: 28.10.1991 DE 4135473
(43) Veröffentlichungstag der Anmeldung: 05.05.1993
(73) Patentinhaber: BAYER AG, 51368 Leverkusen (DE)
(72) Erfinder: Heine, Hans-Georg, Dr., W-4150 Krefeld (DE); Schohe-Loop, Rudolf, Dr., W-5600 Wuppertal 11 (DE); Glaser, Thomas, Dr., W-5063 Overath 3 (DE); De Vry, Jean Marie Viktor, Dr., W-5064 Rösrath (DE); Dompert, Wolfgang, Dr., W-5000 Köln 91 (DE); Sommermeyer, Henning, Dr., W-5000 Köln 80 (DE)

(56) Entgegenhaltungen:
- US-A- 3 845 060
- EUROPEAN JOURNAL OF MEDICINAL CHEMISTRY.CHIMICA THERAPEUTICA. Bd. 9, Nr. 2, M rz 1974, CHATENAY-MALABRY FR Seiten 128 - 132 J. MAILLARD ET AL. 'Composés cycloalcanespiro-hétérocycliques XI. Nouveaux oxo-2 oxa-1 diaza-3,8 spiro(4, 5)décanes substitués en 8, doués de propriétés adrénolytiques'

## Beschreibung

Die Erfindung betrifft 1,3,8-Triazaspiro(4,5)decan-4-on-2-methylchromane, Verfahren zu ihrer Herstellung sowie ihre Verwendung in Arzneimittel, insbesondere als Mittel zur Bekämpfung von Erkrankungen des zentralen Nervensystems.

Aus der DE 21 65 276 sind 1,3,8-Triazaspiro[4,5]decan-4-on substituierte 2-Methyl-benzofuranyle bekannt Außerdem werden im US-Patent 3 826 835 8-Benzofurylmethyl-1,3,8-triazaspiro(4,5)decane als Neuroleptika beschrieben.

Die Erfindung betrifft 1,3,8-Triazaspiro(4,5)decan-4-on-2-methylchromane der allgemeinen Formel (I) 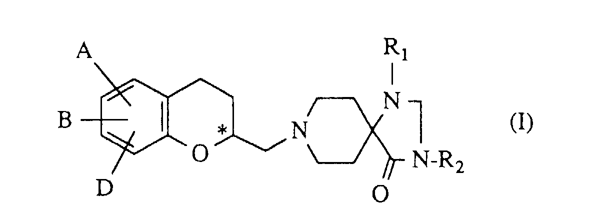 in welcher
- A, B und D: gleich oder verschieden sind und
für Wasserstoff, Halogen, Cyano, Azido, Nitro, Difluormethyl, Trifluormethyl, Difluormethoxy, Trifluormethoxy, Hydroxy oder Carboxy stehen,
oder
für geradkettiges oder verzweigtes Alkyl, Alkenyl, Acyl oder Alkoxycarbonyl mit jeweils bis zu 8 Kohlenstoffatomen stehen, oder für eine Gruppe der Formel -NR³R⁴, -NR⁵-L-R⁶ oder -OR⁷ stehen,
worin
- R³, R⁴ und R⁵: gleich oder verschieden sind und Wasserstoff, geradkettiges oder verzweigtes Alkyl mit bis zu 8 Kohlenstoffatomen, Phenyl oder Benzyl bedeuten,
- L: die -CO- oder -SO₂-Gruppe bedeutet,
- R⁶: geradkettiges oder verzweigtes Alkyl mit bis zu 8 Kohlenstoffatomen oder Benzyl bedeutet, oder
Aryl mit 6 bis 10 Kohlenstoffatomen bedeutet, das gegebenenfalls durch Halogen, Hydroxy, Nitro, Cyano, Trifluormethyl, Trifluormethoxy oder durch geradkettiges oder verzweigtes Alkyl oder Alkoxy mit jeweils bis zu 6 Kohlenstoffatomen substituiert ist,
- R⁷: geradkettiges oder verzweigtes Alkyl oder Alkenyl mit jeweils bis zu 8 Kohlenstoffatomen bedeutet, die gegebenenfalls durch Cycloalkyl mit 3 bis 6 Kohlenstoffatomen oder Phenyl substituiert sind
oder
- A: eine der oben aufgeführten Bedeutungen hat
und
- B und D: gemeinsam einen 5- bis 7-gliedrigen gesättigten, partiell ungesättigten oder aromatischen Carbocyclus oder Heterocyclus mit bis zu 2 Heteroatomen aus der Reihe S, N oder O ausbilden, wobei diese gegebenenfalls bis zu 2 Carbonylfunktionen im Ring besitzen können und die gegebenenfalls bis zu 2-fach gleich oder verschieden durch geradkettiges oder verzweigtes Alkyl oder Alkoxy mit jeweils bis zu 6 Kohlenstoffatomen, Hydroxy, Cycloalkyl mit 3 bis 6 Kohlenstoffatomen, Phenyl, Halogen, Cyano, Nitro oder spiroartig durch einen Rest der Formel 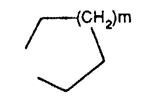 substituiert sind,
worin
- m: eine Zahl 1 oder 2 bedeutet,
und
- R¹ und R²: gleich oder verschieden sind und
für Wasserstoff oder für geradkettiges oder verzweigtes Alkyl stehen, oder für Phenyl oder Benzyl stehen, die gegebenenfalls bis zu 3-fach gleich oder verschieden durch Halogen, Hydroxy, Cyano, Difluormethyl, Difluormethoxy, Trifluormethyl, Trifluormethoxy oder durch geradkettiges oder verzweigtes Alkyl oder Alkoxy mit jeweils bis zu 8 Kohlenstoffatomen substituiert sind,
gegebenenfalls in einer isomeren Form und deren Salze.

Im Rahmen der vorliegenden Erfindung werden physiologisch unbedenkliche Salze bevorzugt. Physiologisch unbedenkliche Salze der substituierten (4,5)decan4-on-2-methylchromane können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Salze im Rahmen der vorliegenden Erfindung sind außerdem Salze der einwertigen Metalle wie Alkalimetalle und die Ammoniumsalze. Bevorzugt werden Natrium-, Kalium- und Ammoniumsalze.

Im Rahmen der vorliegenden Erfindung können die erfindungsgemäßen Verbindungen in verschiedenen stereoisomeren Formen vorliegen. Die erfindungsgemäßen Verbindungen existieren in stereoisomeren Formen, die sich entweder wie Bild un.d Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962].

Bevorzugt sind Verbindungen der allgemeinen Formel (I),
in welcher
- A, B und D: gleich oder verschieden sind und
für Wasserstoff, Fluor, Chlor, Brom, Cyano, Trifluormethyl, Difluormethoxy, Trifluormethoxy oder Hydroxy stehen, oder für geradkettiges oder verzweigtes Alkyl, Alkenyl, Acyl oder Alkoxycarbonyl mit jeweils bis zu 6 Kohlenstoffatomen stehen, oder
für eine Gruppe der Formel -NR³R⁴, -NR⁵-L-R⁶⁵ oder -OR⁷ stehen,
worin
- R³, R⁴ und R⁵: gleich oder verschieden sind und Wasserstoff oder geradkettiges oder verzweigtes Alkyl mit bis zu 6 Kohlenstoffatomen bedeuten,
- L: die -CO- oder -SO₂-Gruppe bedeutet
- R⁶: geradkettiges oder verzweigtes Alkyl mit bis zu 6 Kohlenstoffatomen oder Benzyl bedeutet, oder
Phenyl bedeutet, das gegebenenfalls durch Fluor, Chlor, Brom, Trifluormethyl, Trifluormethoxy, Hydroxy oder durch geradkettiges oder verzweigtes Alkyl oder Alkoxy mit jeweils bis zu 4 Kohlenstoffatomen substituiert ist,
- R⁷: geradkettiges oder verzweigtes Alkyl oder Alkenyl mit bis zu 6 Kohlenstoffatomen bedeutet, die gegebenenfalls durch Cyclopropyl, Cyclopentyl, Cyclohexyl oder Phenyl substituiert sind,
oder
- A: eine der oben aufgeführten Bedeutungen hat
und
- B und D: gemeinsam einen Rest der Formel 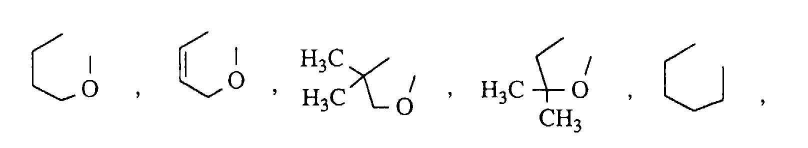 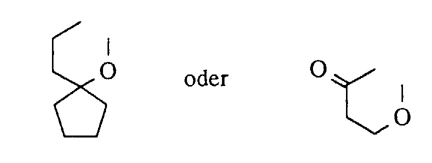 bilden,
- R¹ und R²: gleich oder verschieden sind und
für Wasserstoff oder für geradkettiges oder verzweigtes Alkyl mit bis zu 6 Kohlenstoffatomen stehen, oder
für Phenyl oder Benzyl stehen, die gegebenenfalls bis zu 2-fach gleich verschieden durch Fluor, Chlor, Brom, Hydroxy, Cyano, Trifluormethyl, Trifluormethoxy oder durch geradkettiges oder verzweigtes Alkyl oder Alkoxy mit jeweils bis zu 6 Kohlenstoffatomen substituiert sind
gegebenenfalls in einer isomeren Form und deren Salze.

Besonders bevorzugt sind Verbindungen der allgemeinen Formel (I)
in welcher
- A, B und D: gleich oder verschieden sind und
für Wasserstoff, Fluor, Chlor, Brom, Cyano, Trifluormethyl, Trifluormethoxy oder Hydroxy stehen,
für geradkettiges oder verzweigtes Alkyl oder Alkenyl mit jeweils bis zu 4 Kohlenstoffatomen stehen,
für eine Gruppe der Formel -NR²R³ oder -OR⁶ stehen,
worin
- R² und R³: gleich oder verschieden sind und Wasserstoff oder geradkettiges oder verzweigtes Alkyl mit bis zu 4 Kohlenstoffatomen bedeuten,
- R⁶: geradkettiges oder verzweigtes Alkyl oder Alkenyl mit bis zu 4 Kohlenstoffatomen bedeutet, die gegebenenfalls durch Cyclopropyl oder Phenyl substituiert sind,
oder
- A: eine der oben aufgeführten Bedeutungen hat
und
- B und D: gemeinsam einen Rest der Formel 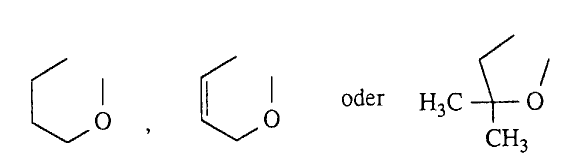 bilden,
- R¹ und R²: gleich oder verschieden sind und
für Wasserstoff oder für geradkettiges oder verzweigtes Alkyl mit bis zu 4 Kohlenstoffatomen stehen, oder
für Phenyl oder Benzyl stehen, die gegebenenfalls durch Fluor, Chlor, Brom, Hydroxy, Trifluormethyl, Trifluormethoxy oder durch geradkettiges oder
verzweigtes Alkyl oder Alkoxy mit jeweils bis zu 4 Kohlenstoffatomen substituiert sind
gegebenenfalls in einer isomeren Form und deren Salze.

Ganz besonders bevorzugt sind Verbindungen der allgemeinen Formel (I),
in welcher
- A, B, D: für Wasserstoff oder für Methoxy, R¹ für Phenyl und R² für Wasserstoff stehen.

Außerdem wurde ein Verfahren zur Herstellung der erfindungsgemäßen Verbindungen der allgemeinen Formel (I) gefunden, dadurch gekennzeichnet, daß man
Verbindungen der allgemeinen Formel (II) 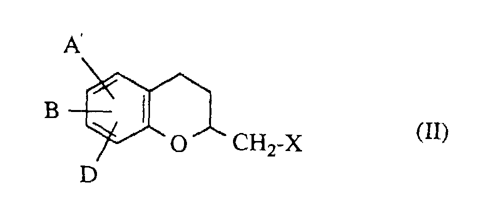 in welcher
- A, B und D: die oben angegebene Bedeutung haben
und
- X: für Hydroxy oder für eine typische Abgangsgruppe, wie Tosyloxy, Mesyloxy, Chlor oder Brom steht,
in inerten Lösemitteln, in Anwesenheit einer Base und gegebenenfalls eines Katalysators mit Verbindungen der allgemeinen Formel (III) 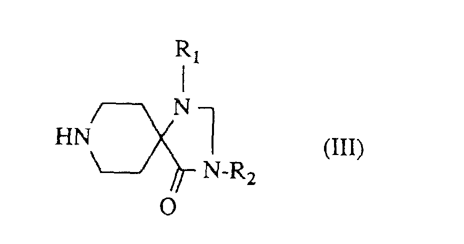 in welcher
- R¹ und R²: die oben angegebene Bedeutung haben,
umsetzt und gegebenenfalls die Substituenten A, B und D nach üblicher Methode variiert.

Das erfindungsgemäße Verfahren kann durch folgendes Formelschema beispielhaft erläutert werden:

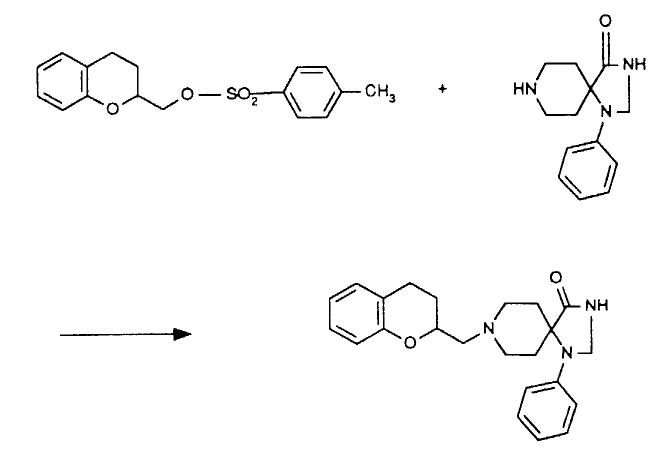

Als Lösemittel eignen sich die üblichen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether oder Butylmethylether, oder Ketone wie Aceton oder Butanon, oder Amide wie Dimethylformamid oder Hexamethylphosphorsäuretriamid, oder Dimethylsulfoxid, Acetonitril, Essigester, oder Halogenkohlenwasserstoffe wie Methylenchlorid, Chloroform oder Tetrachlorkohlenstoff, oder Pyridin, Picolin oder N-Methylpiperidin. Ebenso können Gemische der genannten Lösemittel verwendet werden. Bevorzugt ist Dimethylformamid.

Als Basen eignen sich die üblichen anorganischen oder organischen Basen. Hierzu gehören bevorzugt Alkalihydroxide wie beispielsweise Natrium- oder Kaliumhydroxid, oder Alkalicarbonate wie Natrium- oder Kaliumcarbonat, oder Alkalialkoholate wie beispielsweise Natrium- oder Kaliummethanolat, oder Natrium- oder Kaliumethanolat, oder organische Amine wie Triethylamin, Picolin oder N-Methylpiperidin, oder Amide wie Natriumamid oder Lithiumdiisopropylamid, oder metallorganische Verbindungen wie Butyllithium oder Phenyllithium. Bevorzugt sind Natrium- und Kaliumcarbonat und Triethylamin.

Die Base wird in einer Menge von 0,6 mol bis 5 mol, bevorzugt von 0,7 mol bis 2 mol bezogen auf 1 mol der Verbindung der allgemeinen Formel (II) eingesetzt.

Die Reaktion wird im allgemeinen in einem Temperaturbereich von 0°C bis 150°C, bevorzugt von +20°C bis +110°C durchgeführt.

Die Umsetzung kann bei normalem, erhöhtem oder bei erniedrigtem Druck durchgeführt werden (z.B. 0,5 bis 5 bar). Im allgemeinen arbeitet man bei Normaldruck.

Als Katalysatoren eignen sich im allgemeinen Alkalimetallhalogenide wie beispielsweise Natrium- oder Kaliumjodid. Bevorzugt ist Natriumjodid.

Der Katalysator wird im allgemeinen in einer Menge von 0,05 - 1,0 mol, bevorzugt von 0,1 bis 0,5 mol, bezogen auf 1 mol der Verbindungen der allgemeinen Formel (II) eingesetzt.

Die Verbindungen der allgemeinen Formel (II) sind bekannt oder können nach üblicher Methode hergestellt werden [vgl. US 4 957 928; Farmaco, Ed. Sci. 42 (11), 805-13; Eur. J. Med. Chem. 22 (6), 539 - 44; EP 252 005; EP 199 400; Eur. J. Med. Chem.-Chim. Ther. 20 (2), 117 - 20; Nouv. J. Chim. 6 (3), 149-154].

Ebenso sind die Verbindungen der allgemeinen Formel (III) bekannt [vgl. US 3 826 835]; [CAS, 1021-25-6].

Die erfindungsgemäßen Verbindungen können als Wirkstoffe in Arzneimitteln verwendet werden. Die erfindungsgemäßen Stoffe haben eine besonders hohe Affinität zu cerebralen 5-Hydroxy-tryptamin-Rezeptoren vom 5-HT₁-Typ. Auch besitzen sie hohe Affinität an Dopamin-Rezeptoren vom D₂-Typ.

Die erfindungsgemäßen Stoffe zeigen überraschenderweise eine vorteilhafte Wirkung auf das Zentralnervensystem und können zur therapeutischen Behandlung von Menschen und Tieren verwendet werden.

Die in der vorliegenden Erfindung beschriebenen Verbindungen stellen somit Wirkstoffe zur Bekämpfung von Krankheiten dar, die durch Störungen des serotoninergen und dopaminergen Systems, insbesondere bei Involvierung von Rezeptoren, die hohe Affinität zu 5-Hydroxytryptamin (5-HT₁-Typ) und/oder zu Dopamin (D₂-Typ) besitzen, gekennzeichnet sind. Sie eignen sich daher zur Behandlung von Erkrankungen des Zentralnervensystems wie Angst-, Spannungs-und Depressionszuständen, zentralnervös bedingten Sexualdysfunktionen und Schlafstörungen, sowie zur Regulierung krankhafter Störungen der Nahrungs-, Genuß- und Suchtmittelaufnahme. Weiterhin sind sie geeignet zur Beseitigung kognitiver Deficite, zur Verbesserung von Lern- und Gedächtnisleistungen und zur Behandlung der Alzheimer'schen Krankheit. Auch sind sie geeignet zur Bekämpfung von Psychosen (z.B- Schizophrenie, Manie). Gegenüber bekannter Neuroleptika besitzen sie ein geringeres Nebenwirkungspotential.

Weiterhin eignen sich diese Wirkstoffe auch zur Modulierung des cardiovaskulären Systems. Sie greifen auch in die Regulation der cerebralen Durchblutung ein und stellen somit wirkungsvolle Mittel zur Bekämpfung von Migräne dar.

Auch eignen sie sich zur Prophylaxe und Bekämpfung der Folgen cerebraler Infarktgeschehen (Apoplexia cerebri) wie Schlaganfall, cerebraler Ischämien. Darüberhinaus können die Verbindungen zur Behandlung von akutem Schädel-Hirn-Trauma verwendet werden. Ebenso können die erfindungsgemäßen Verbindungen zur Bekämpfung von Schmerzzuständen eingesetzt werden.

### Affinität zum 5-HT₁-Rezeptor

In Tabelle 1 wird beispielhaft die hohe Affinität der erfindungsgemäßen Verbindungen zu 5-Hydroxytryptamin-Rezeptoren vom Subtyp 1 dargestellt. Bei den angegebenen Werten handelt es sich um Daten, die aus Rezeptorbindungsstudien mit Kalbs-Hippocampus-Membranpräparationen ermittelt wurden. Als radioaktiv markierter Ligand wurde hierzu ³H-Serotonin verwendet.

**Tabelle [A]**

| Verbindung des Beispiels | Kᵢ (nmol/l) |
|---|---|
| 1 | 2 |
| 2 | 1,5 |
| 4 | 1 |

### Affinität zum 5-HT_{1A}-Rezeptor

[W.U. Dompert et al., Naunyn-Schmiedeberg's Arch. Pharmacol. (1985), 328, 467-470].

Bei diesem Test wird die Bindung von ³H-Ipsapiron an 5-HT_{1A}-Rezeptoren in Kalbshippocampus-Membranen gemessen. Es wurde gefunden, daß die erfindungsgemäßen Verbindungen mit dem Radioliganden um die Bindung konkurrieren und diese hemmen.

**Tabelle [B]**

| Verbindung des Beispiels | Kᵢ (nmol/l) |
|---|---|
| 5 | 1 |
| 6 | 1 |

### Dopamin D₂-Rezeptortest

Dieser Test wird nach folgender Literaturstelle durchgeführt: Imafuku J. (1987), Brain Research 402; 331-338.

Dabei wird die Bindung des selektiven D₂-Rezeptor-Antagonisten ³H-Sulpirid an Membranen aus dem Striatum der Ratte gemessen. Verbindungen, die an Dopamin-D₂-Rezeptoren binden, hemmen konzentrationsabhängig die Bindung von ³H-Sulpirid. Aus den Verdrängungskurven werden IC₅₀-Werte ermittelt und daraus die Inhibitionskonstanten Kᵢ berechnet.

**Tabelle [C]**

| Verbindung des Beispiels | Kᵢ (nmol/l) |
|---|---|
| 1 | 0,2 |
| 2 | 0,3 |
| 3 | 0,6 |

Zur vorliegenden Erfindung gehören auch pharmazeutische Zubereitungen, die neben inerten, nicht-toxischen, pharmazeutisch geeigneten Hilfs- und Trägerstoffen eine oder mehrere Verbindungen der allgemeinen Formel (I) enthalten, oder die aus einem oder mehreren Wirkstoffen der Formel (I) bestehen, sowie Verfahren zur Herstellung dieser Zubereitungen.

Die Wirkstoffe der Formel (I) sollen in diesen Zubereitungen in einer Konzentration von 0,1 bis 99,5 Gew.-%, bevorzugt von 0,5 bis 95 Gew.-% der Gesamtmischung vorhanden sein.

Neben den Wirkstoffen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den oder die Wirkstoffe der Formel (I) in Gesamtmengen von etwa 0,01 bis etwa 100 mg/kg, bevorzugt in Gesamtmengen von etwa 0,1 mg/kg bis 5 mg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und vom Körpergewicht des behandelten Objekts, vom individuellen Verhalten gegenüber dem Medikament, der Art und Schwere der Erkrankung, der Art der Zubereitung und Applikation, sowie dem Zeitpunkt bzw. Intervall, zu welchem die Verabreichung erfolgt.

Die jeweils aufgeführten R_{f}-Werte wurden - sofern nicht anders vermerkt - durch Dünnschichtchromatographie auf Kieselgel (Alufolie, Kieselgel 60 F 254, Fa. E. Merck) ermittelt. Die Visualisierung der Substanzflecke geschah durch Betrachten unter UV-Licht und/oder durch Besprühen mit 1%iger Kaliumpermanganat-Lösung.

Die Flash-Chromatographie wurde auf Kieselgel 60, 0,040 - 0,064 mm, Fa. E. Merck, durchgeführt (siehe Still et al., J. Org. Chem. 43, 2923, 1978; für einfachere Trennprobleme siehe Aldrichimica Acta 18, 25, 1985). Elution mit Lösemittelgradienten bedeutet: Beginnend mit der reinen, unpolaren Lösemittelgemischkomponente wird in einem steigendem Ausmaß die polare Laufmittelkomponente zugemischt, bis das gewünschte Produkt eluiert wird (DC-Kontrolle).

Bei allen Produkten wurde bei zuletzt ca. 13 Pa (0,1 Torr) das Lösemittel abdestilliert. Salze wurden bei diesem Druck über Nacht über Kaliumhydroxid und/oder Phosphorpentoxid aufbewahrt.

### Ausgangsverbindungen

### Beispiel I

### 2-Hydroxymethyl-8-methoxy-chroman

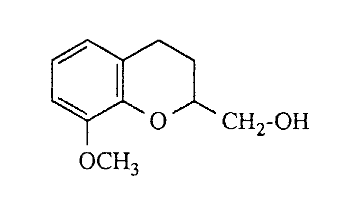

59,0 g (0,25 mol) 8-Methoxy-chroman-2-carbonsäureethylester werden in 525 ml wasserfreiem Tetrahydrofuran innerhalb 1 h unter Rühren bei 20°C zu der Suspension von 9,5 g (0,25 mol) Lithiumaluminiumhydrid in 525 ml wasserfreiem Diethylether getropft. Der Ansatz wird über Nacht gerührt und anschließend unter Kühlen nacheinander mit 9,5 ml Wasser, 9,5 ml 15%iger Natronlauge und 28,4 ml Wasser tropfenweise versetzt. Die organische Phase wird dekantiert und eingedampft. Der Rückstand wird 2 mal aus Dichlormethan / Petrolether umkristallisiert.
Ausbeute: 38,0 g (87%)
Schmp.: 57-58°C

### Beispiel II

### (2R)-2-Hydroxymethyl-chroman

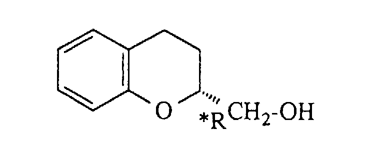

Zu der Lösung von 22,1 g (0,124 mol) (2R)-Chroman-2-carbonsäure (ee = 98,3%) in 210 ml wasserfreiem Tetrahydrofuran unter Argon werden innerhalb von 30 Minuten bei 0°C Innentemperatur 164 ml einer 1 M Boranlösung in Tetrahydrofuran zugetropft. Die Kühlung wird entfernt, und der Ansatz 4 h nachgerührt. Die Innentemperatur steigt währenddessen auf 34°C an. Anschließend werden 46 ml eines 1/1-Gemisches aus Tetrahydrofuran und Wasser unter Eiskühlung zugetropft. Nach Zugabe von 40,7 g wasserfreiem Kaliumcarbonat und kräftigem Rühren wird die Tetrahydrofuranlösung dekantiert und im Wasserstrahlvakuum eingeengt. Kurzwegdestillation liefert 18,8 g farbloses 2R-Hydroxymethylchroman vom Sdp. 77-78°C/0,15 mbar.
ee >99%.

### Beispiel III

### (2S)-2-Hydroxymethyl-chroman

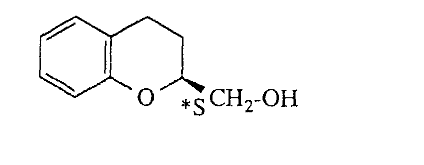

In Analogie zur Vorschrift des Beispiels II wird die Titelverbindung aus (2S)-2-Chroman-2-carbonsäure hergestellt.
ee>99%
Kp.:79-81°C/0,15 mbar

### Beispiel IV

### (2R)-2-Tosyloxymethyl-chroman

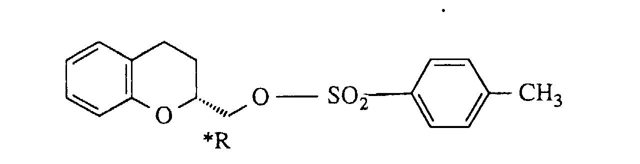

Zu 12,8 g (0,78 mol) (2R)-2-Hydroxymethylchroman (Beispiel II) in 50 ml wasserfreiem Pyridin werden unter Rühren und Eiskühlung 15,63 g 4-Toluolsulfochlorid portionsweise gegeben. Nach Stehenlassen über Nacht wird der Ansatz in Eiswasser eingetragen und mit Diethylether extrahiert Die Etherphase wird 2 mal mit 5%iger eiskalter Salzsäure und anschließend mit gesättigter Kochsalzlösung gewaschen, über wasserfreiem Natriumsulfat getrocknet und im Wasserstrahlvakuum eingedampft. 22,4 g einheitlicher 4-Toluolsulfonsäureester des 2R-2-Hydroxymethylchromans werden erhalten.
R_{f} = 0,6 (Toluol / Essigester 3:1) Öl, [α]_{D} = 51,1° (c = 1, CHCl₃) F°C = 61,5-64,5 (aus Dichlormethan/Petrolether).

### Beispiel V

### (2S)-2-Tosyloxymethyl-chroman

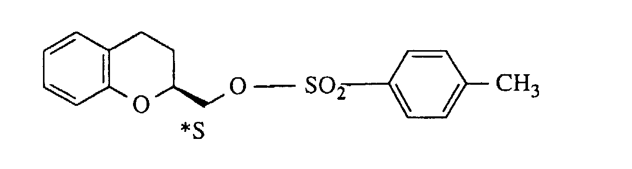

In Analogie zur Vorschrift des Beipsiels IV wird die Titelverbindung aus Beispiel III hergestellt
R_{f} = 0,6 (Toluol / Essigester 3:1) Öl

### Beispiel VI

### 8-Methoxy-2-tosyloxymethyl-chroman

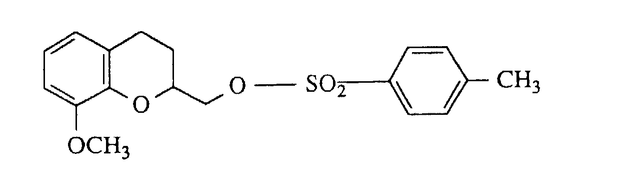

Schmp.: 115-117 (aus Dichlormethan)

### Herstellungsbeispiele

### Beispiel 1

### 8-(Chroman-2-yl-methyl)-1-phenyl-1,3,8-triazaspiro(4,5)decan-4-on

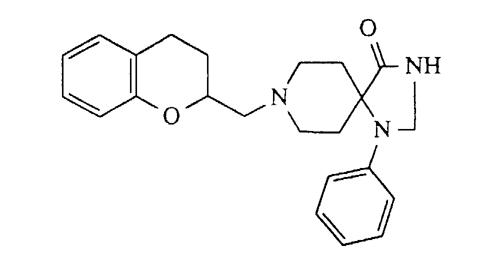

Das Gemisch aus 31,8 g (0,1 mol) 2-Tosyloxymethyl-chroman, 7,1 g (0,07 mol) wasserfreiem Natriumcarbonat und 23,1 g (0,1 mol) 1-Phenyl-1,3,8-triazaspiro[4,5]-decan-4-on in 240 ml wasserfreiem Dimethylformamid (DMF) wird 6 h bei 110°C gerührt und anschließend auf Eis (500 g) gegossen. Nach Extrahieren mit Essigsäureethylester (5 x 100 ml), Waschen der organischen Extrakte mit Wasser, Trocknen über wasserfreiem Natriumsulfat und Eindampfen der organischen Phase im Wasserstrahlvakuum werden 65,7 g lösemittelhaltiges kristallines Rohprodukt erhalten, das 2 mal aus Essigsäureethylester umkristallisiert, 20,6 g der Titelverbindung vom Schmp. 192-193,5°C liefert.
Ausbeute: 55% der Theorie

In Analogie zur Vorschrift aus Beispiel 1 werden die in Tabelle 1 aufgeführten Beispiele hergestellt:

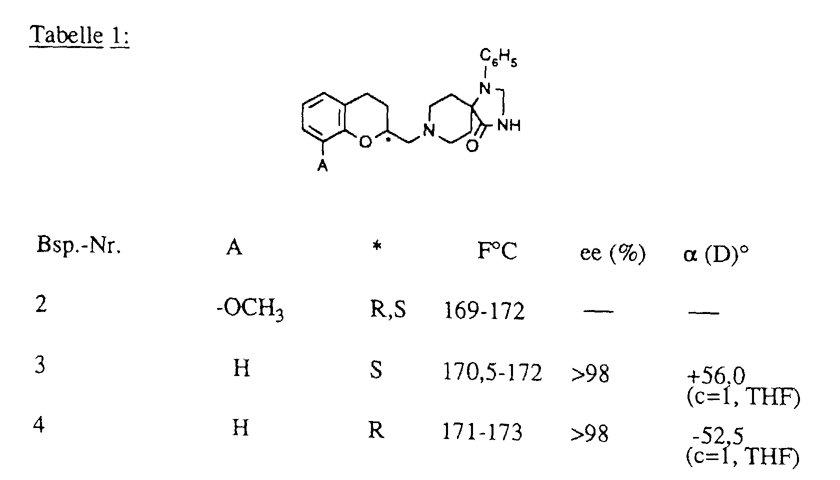

### Beispiel 5

8-(Chroman-2-yl-methyl)-1-phenyl-1,3,8-triazaspiro(4,5)decan-4-on HCl-Salz 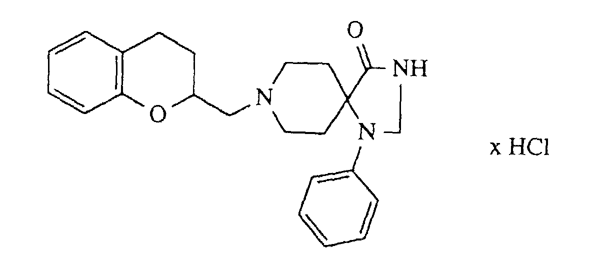 3,34 g (0,01 mol) 8-(Chroman-2-yl-methyl)-1-phenyl-1,3,8-triazaspiro(4,5)-decan-4-on werden in 100 ml Diethylether unter Zusatz von 20 ml Dichlormethan gelöst und unter Rühren mt 6,9 ml 1,45 n etherischer Chlorwasserstoffsäure unter Eiskühlung versetzt. Nach 2 h wird der Niederschlag abgesaugt, mit Diethylether gewaschen und im Ölpumpenvakuum bei 60°C getrocknet 3,4 g der Titelverbindung vom Schmp. 238-240°C (Kap.) werden erhalten.

In Analogie zur Vorschrift des Beispiels 5 werden die in Tabelle 2 aufgeführten Verbindungen hergestellt:

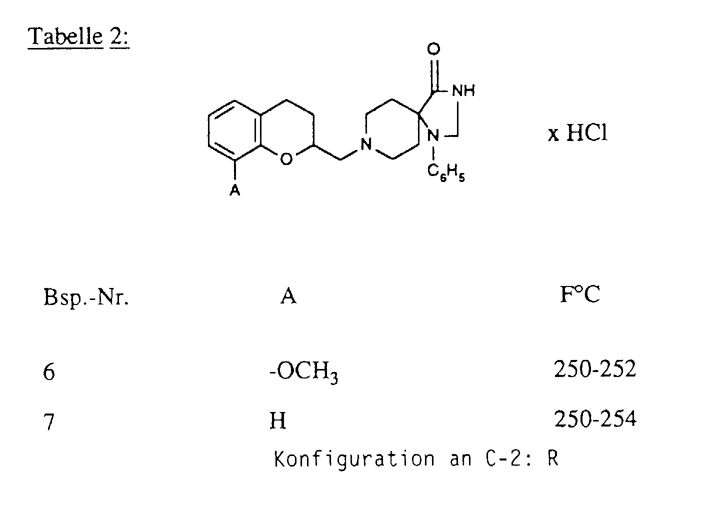

## Patentansprüche

1. Triazaspirodecanon-methylchromane der allgemeinen Formel 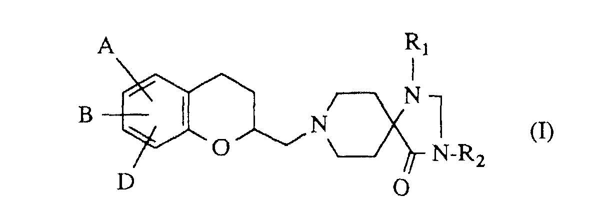 in welcher
A, B und D gleich oder verschieden sind und
für Wasserstoff, Halogen, Cyano, Azido, Nitro, Difluormethyl, Trifluormethyl, Difluormethoxy, Trifluormethoxy, Hydroxy oder Carboxy stehen, oder
für geradkettiges oder verzweigtes Alkyl, Alkenyl, Acyl oder Alkoxycarbonyl mit jeweils bis zu 8 Kohlenstoffatomen stehen, oder für eine Gruppe der Formel -NR³R⁴, -NR⁵-L-R⁶ oder -OR⁷ stehen,
worin
R³, R⁴ und R⁵ gleich oder verschieden sind und Wasserstoff, geradkettiges oder verzweigtes Alkyl mit bis zu 8 Kohlenstoffatomen, Phenyl oder Benzyl bedeuten,
L die -CO- oder -SO₂-Gruppe bedeutet,
R⁶ geradkettiges oder verzweigtes Alkyl mit bis zu 8 Kohlenstoffatomen oder Benzyl bedeutet, oder
Aryl mit 6 bis 10 Kohlenstoffatomen bedeutet, das gegebenenfalls durch Halogen, Hydroxy, Nitro, Cyano, Trifluormethyl, Trifluormethoxy oder durch geradkettiges oder verzweigtes Alkyl oder Alkoxy mit jeweils bis zu 6 Kohlenstoffatomen substituiert ist,
R⁷ geradkettiges oder verzweigtes Alkyl oder Alkenyl mit jeweils bis zu 8 Kohlenstoffatomen bedeutet, die gegebenenfalls durch Cycloalkyl mit 3 bis 6 Kohlenstoffatomen oder Phenyl substituiert sind
oder
A eine der oben aufgeführten Bedeutungen hat
und
B und D gemeinsam einen 5- bis 7-gliedrigen gesättigten, partiell ungesättigten oder aromatischen Carbocyclus oder Heterocyclus mit bis zu 2 Heteroatomen aus der Reihe S, N oder O ausbilden, wobei diese gegebenenfalls bis zu 2 Carbonylfunktionen im Ring besitzen können und die gegebenenfalls bis zu 2-fach gleich oder verschieden durch geradkettiges oder verzweigtes Alkyl oder Alkoxy mit jeweils bis zu 6 Kohlenstoffatomen, Hydroxy, Cycloalkyl mit 3 bis 6 Kohlenstoffatomen, Phenyl, Halogen, Cyano, Nitro oder spiroartig durch einen
Rest der Formel 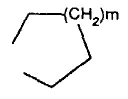 substituiert sind,
worin
m eine Zahl 1 oder 2 bedeutet,
und
R¹ und R² gleich oder verschieden sind und
für Wasserstoff oder für geradkettiges oder verzweigtes Alkyl stehen, oder
für Phenyl oder Benzyl stehen, die gegebenenfalls bis zu 3-fach gleich oder verschieden durch Halogen, Hydroxy, Cyano, Difluormethyl, Difluormethoxy, Trifluormethyl, Trifluormethoxy oder durch geradkettiges oder verzweigtes Alkyl oder Alkoxy mit jeweils bis zu 8 Kohlenstoffatomen substituiert sind,
gegebenenfalls in einer isomeren Form und deren Salze.

2. Triazaspirodecanon-methylchromane nach Anspruch 1
wobei
A, B und D gleich oder verschieden sind und
für Wasserstoff, Fluor, Chlor, Brom, Cyano, Trifluormethyl, Difluormethoxy, Trifluormethoxy oder Hydroxy stehen, oder
für geradkettiges oder verzweigtes Alkyl, Alkenyl, Acyl oder Alkoxycarbonyl mit jeweils bis zu 6 Kohlenstoffatomen stehen, oder für eine Gruppe der Formel -NR³R⁴, -NR⁵-L-R⁶⁵ oder -OR⁷ stehen,
worin
R³, R⁴ und R⁵ gleich oder verschieden sind und Wasserstoff oder geradkettiges oder verzweigtes Alkyl mit bis zu 6 Kohlenstoffatomen bedeuten,
L die -CO- oder -SO₂-Gruppe bedeutet
R⁶ geradkettiges oder verzweigtes Alkyl mit bis zu 6 Kohlenstoffatomen oder Benzyl bedeutet, oder
Phenyl bedeutet, das gegebenenfalls durch Fluor, Chlor, Brom, Trifluormethyl, Trifluormethoxy, Hydroxy oder durch geradkettiges oder verzweigtes Alkyl oder Alkoxy mit jeweils bis zu 4 Kohlenstoffatomen substituiert ist,
R⁷ geradkettiges oder verzweigtes Alkyl oder Alkenyl mit bis zu 6 Kohlenstoffatomen bedeutet, die gegebenenfalls durch Cyclopropyl, Cyclopentyl, Cyclohexyl oder Phenyl substituiert sind,
oder
A eine der oben aufgeführten Bedeutungen hat
und
B und D gemeinsam einen Rest der Formel 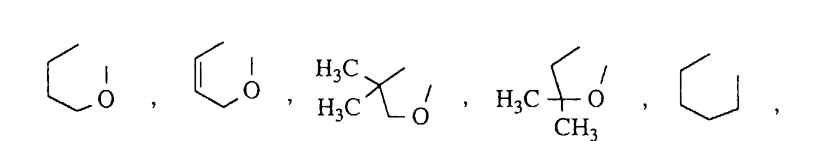 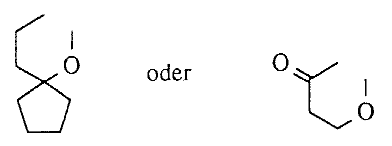 bilden,
R¹ und R² gleich oder verschieden sind und
für Wasserstoff oder für geradkettiges oder verzweigtes Alkyl mit bis zu 6 Kohlenstoffatomen stehen, oder
für Phenyl oder Benzyl stehen, die gegebenenfalls bis zu 2-fach gleich verschieden durch Fluor, Chlor, Brom, Hydroxy, Cyano, Trifluormethyl, Trifluormethoxy oder durch geradkettiges oder verzweigtes Alkyl oder Alkoxy mit jeweils bis zu 6 Kohlenstoffatomen substituiert sind
gegebenenfalls in einer isomeren Form und deren Salze.

3. Triazaspirodecanon-methylchromane nach Anspruch 1
wobei
A, B und D gleich oder verschieden sind und
für Wasserstoff, Fluor, Chlor, Brom, Cyano, Trifluormethyl, Trifluormethoxy oder Hydroxy stehen,
für geradkettiges oder verzweigtes Alkyl oder Alkenyl mit jeweils bis zu 4 Kohlenstoffatomen stehen,
für eine Gruppe der Formel -NR²R³ oder -OR⁶ stehen,
worin
R² und R³ gleich oder verschieden sind und Wasserstoff oder geradkettiges oder verzweigtes Alkyl mit bis zu 4 Kohlenstoffatomen bedeuten,
R⁶ geradkettiges oder verzweigtes Alkyl oder Alkenyl mit bis zu 4 Kohlenstoffatomen bedeutet, die gegebenenfalls durch Cyclopropyl oder Phenyl substituiert sind,
oder
A eine der oben aufgeführten Bedeutungen hat
und
B und D gemeinsam einen Rest der Formel 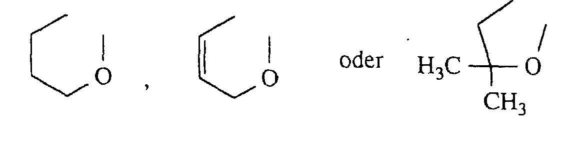 bilden,
R¹ und R² gleich oder verschieden sind und
für Wasserstoff oder für geradkettiges oder verzweigtes Alkyl mit bis zu 4 Kohlenstoffatomen stehen, oder
für Phenyl oder Benzyl stehen, die gegebenenfalls durch Fluor, Chlor, Brom, Hydroxy, Trifluormethyl, Trifluormethoxy oder durch geradkettiges oder verzweigtes Alkyl oder Alkoxy mit jeweils bis zu 4 Kohlenstoffatomen substituiert sind
gegebenenfalls in einer isomeren Form und deren Salze.

4. Triazaspirodecanon-methylchromane nach Anspruch 1 zur Behandlung von Krankheiten.

5. Verfahren zur Herstellung von Triazaspirodecanon-Methylchromane der allgemeinen Formel 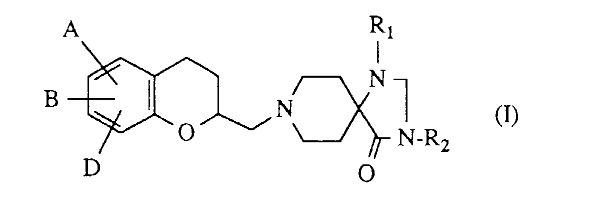 in welcher
A, B und D gleich oder verschieden sind und
für Wasserstoff, Halogen, Cyano, Azido, Nitro, Difluormethyl, Trifluormethyl, Difluormethoxy, Trifluormethoxy, Hydroxy oder Carboxy stehen, oder
für geradkettiges oder verzweigtes Alkyl, Alkenyl, Acyl oder Alkoxycarbonyl mit jeweils bis zu 8 Kohlenstoffatomen stehen, oder für eine Gruppe der Formel -NR³R⁴, -NR⁵-L-R⁶ oder -OR⁷ stehen,
worin
R³, R⁴ und R⁵ gleich oder verschieden sind und Wasserstoff, geradkettiges oder verzweigtes Alkyl mit bis zu 8 Kohlenstoffatomen, Phenyl oder Benzyl bedeuten,
L die -CO- oder -SO₂-Gruppe bedeutet,
R⁶ geradkettiges oder verzweigtes Alkyl mit bis zu 8 Kohlenstoffatomen oder Benzyl bedeutet, oder
Aryl mit 6 bis 10 Kohlenstoffatomen bedeutet, das gegebenenfalls durch Halogen, Hydroxy, Nitro, Cyano, Trifluormethyl, Trifluormethoxy oder durch geradkettiges oder verzweigtes Alkyl oder Alkoxy mit jeweils bis zu 6 Kohlenstoffatomen substituiert ist,
R⁷ geradkettiges oder verzweigtes Alkyl oder Alkenyl mit jeweils bis zu 8 Kohlenstoffatomen bedeutet, die gegebenenfalls durch Cycloalkyl mit 3 bis 6 Kohlenstoffatomen oder Phenyl substituiert sind
oder
A eine der oben aufgeführten Bedeutungen hat
und
B und D gemeinsam einen 5- bis 7-gliedrigen gesättigten, partiell ungesättigten oder aromatischen Carbocyclus oder Heterocyclus mit bis zu 2 Heteroatomen aus der Reihe S, N oder O ausbilden, wobei diese gegebenenfalls bis zu 2 Carbonylfunktionen im Ring besitzen können und die gegebenenfalls bis zu 2-fach gleich oder verschieden durch geradkettiges oder verzweigtes Alkyl oder Alkoxy mit jeweils bis zu 6 Kohlenstoffatomen, Hydroxy, Cycloalkyl mit 3 bis 6 Kohlenstoffatomen, Phenyl, Halogen, Cyano, Nitro oder spiroartig durch einen Rest der Formel 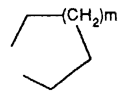 substituiert sind,
worin
m eine Zahl 1 oder 2 bedeutet,
und
R¹ und R² gleich oder verschieden sind und
für Wasserstoff oder für geradkettiges oder verzweigtes Alkyl stehen, oder
für Phenyl oder Benzyl stehen, die gegebenenfalls bis zu 3-fach gleich oder verschieden durch Halogen, Hydroxy, Cyano, Difluormethyl, Difluormethoxy, Trifluormethyl, Trifluormethoxy oder durch geradkettiges oder verzweigtes Alkyl oder Alkoxy mit jeweils bis zu 8 Kohlenstoffatomen substituiert sind,
gegebenenfalls in einer isomeren Form und deren Salze,
dadurch gekennzeichnet, daß man Verbindungen der allgemeinen Formel (II) 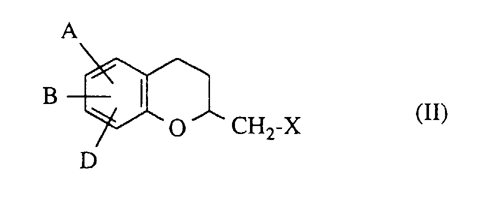 in welcher
A, B und D die oben angegebene Bedeutung haben
und
X für Hydroxy oder für eine typische Abgangsgruppe, wie Tosyloxy, Mesyloxy, Chlor oder Brom steht,
in inerten Lösemitteln, in Anwesenheit einer Base und gegebenenfalls eines Katalysators mit Verbindungen der allgemeinen Formel (III) 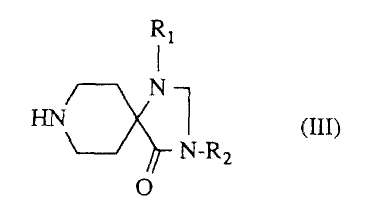 in welcher
R¹ und R² die oben angegebene Bedeutung haben,
umsetzt und gegebenenfalls die Substituenten A, B und D nach üblicher Methode variiert.

6. Verfahren nach Anspruch 5, dadurch gekennzeichnet, daß man mit einer Temperatur von 0°C bis +150°C arbeitet.

7. Arzneimittel enthaltend mindestens ein Triazaspirodecanon-methylchroman nach Anspruch 1.

8. Arzneimittel nach Anspruch 7 zur Behandlung von Erkrankungen, die durch Störungen des serotoninergen und dopaminergen Systems gekennzeichnet sind.

9. Verwendung von Triazaspirodecanon-methylchromanen nach Anspruch 1 zur Herstellung von Arzneimitteln.

10. Verfahren zur Herstellung von Arzneimittel nach Anspruch 7 dadurch gekennzeichnet, daß man die Triazaspirodecanon-methylchromane gegebenenfalls mit Hilfe von üblichen Hilfs- und Trägerstoffen in eine geeignete Applikationsform überführt.

## Claims

1. Triazaspirodecanone-methylchromans of the general formula 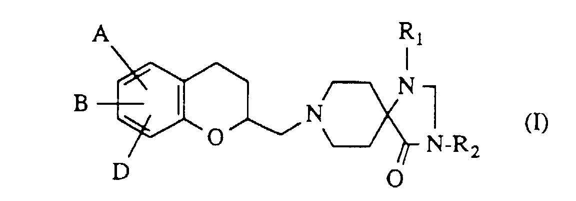 in which
A, B and D are identical or different and
represent hydrogen, halogen, cyano, azido, nitro, difluoromethyl, trifluoromethyl, difluoromethoxy, trifluoromethoxy, hydroxyl or carboxyl, or
represent straight-chain or branched alkyl, alkenyl, acyl or alkoxycarbonyl each having up to 8 carbon atoms, or
represent a group of the formula -NR³R⁴, -NR⁵-L-R⁶ or -OR⁷,
in which
R³, R⁴ and R⁵ are identical or different and denote hydrogen, straight-chain or branched alkyl having up to 8 carbon atoms, phenyl or benzyl,
L denotes the -CO- or -SO₂- group,
R⁶ denotes straight-chain or branched alkyl having up to 8 carbon atoms or benzyl, or denotes aryl having 6 to 10 carbon atoms, which is optionally substituted by halogen, hydroxyl, nitro, cyano, trifluoromethyl, trifluoromethoxy or by straight-chain or branched alkyl or alkoxy each having up to 6 carbon atoms,
R⁷ denotes straight-chain or branched alkyl or alkenyl each having up to 8 carbon atoms, each of which is optionally substituted by cycloalkyl having 3 to 6 carbon atoms or phenyl
or
A has one of the abovementioned meanings
and
B and D together form a 5- to 7-membered saturated, partially unsaturated or aromatic carbocyclic ring or heterocyclic ring having up to 2 heteroatoms from the series comprising S, N and O, where these rings can optionally have up to 2 carbonyl functions in the ring and are optionally monosubstituted or disubstituted by identical or different substituents from the series comprising straight-chain or branched alkyl or alkoxy each having up to 6 carbon atoms, hydroxyl, cycloalkyl having 3 to 6 carbon atoms, phenyl, halogen, cyano, nitro or in spiro fashion by a radical of the formula 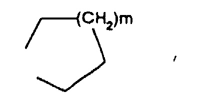 in which
m denotes a number 1 or 2,
and
R¹ and R² are identical or different and
represent hydrogen or straight-chain or branched alkyl, or
represent phenyl or benzyl, each of which is optionally monosubstituted to trisubstituted by identical or different substituents from the series comprising halogen, hydroxyl, cyano, difluoromethyl, difluoromethoxy, trifluoromethyl and trifluoromethoxy or by straight-chain or branched alkyl or alkoxy each having up to 8 carbon atoms,
if appropriate in an isomeric form, and their salts.

2. Triazaspirodecanone-methylchromans according to Claim 1 where
A, B and D are identical or different and
represent hydrogen, fluorine, chlorine, bromine, cyano, trifluoromethyl, difluoromethoxy, trifluoromethoxy or hydroxyl, or
represent straight-chain or branched alkyl, alkenyl, acyl or alkoxycarbonyl each having up to 6 carbon atoms, or
represent a group of the formula -NR³R⁴, -NR⁵-L-R⁶ or -OR⁷,
in which
R³, R⁴ and R⁵ are identical or different and denote hydrogen or straight-chain or branched alkyl having up to 6 carbon atoms,
L denotes the -CO- or -SO₂- group,
R⁶ denotes straight-chain or branched alkyl having up to 6 carbon atoms or benzyl, or denotes phenyl which is optionally substituted by fluorine, chlorine, bromine, trifluoromethyl, trifluoromethoxy, hydroxyl or by straight-chain or branched alkyl or alkoxy each having up to 4 carbon atoms,
R⁷ denotes straight-chain or branched alkyl or alkenyl having up to 6 carbon atoms, each of which is optionally substituted by cyclopropyl, cyclopentyl, cyclohexyl or phenyl,
or
A has one of the abovementioned meanings
and
B and D together form a radical of the formula 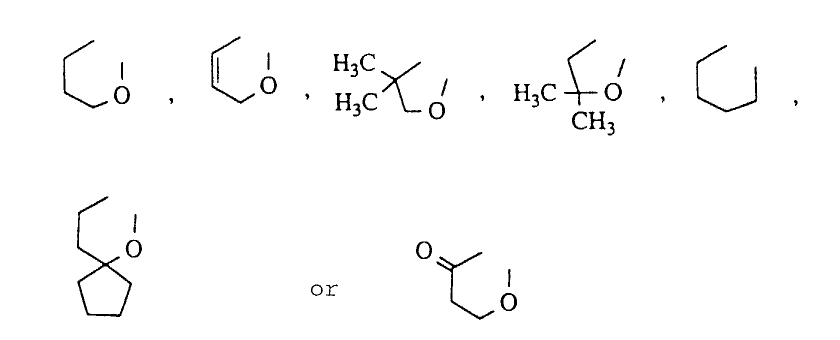
R¹ and R² are identical or different and represent hydrogen or straight-chain or branched alkyl having up to 6 carbon atoms, or represent phenyl or benzyl, each of which is optionally monosubstituted or disubstituted by identical or different substituents from the series comprising fluorine, chlorine, bromine, hydroxyl, cyano, trifluoromethyl and trifluoromethoxy or by straight-chain or branched alkyl or alkoxy each having up to 6 carbon atoms,
if appropriate in an isomeric form, and their salts.

3. Triazaspirodecanone-methylchromans according to Claim 1 in which
A, B and D are identical or different and
represent hydrogen, fluorine, chlorine, bromine, cyano, trifluoromethyl, trifluoromethoxy or hydroxyl,
represent straight-chain or branched alkyl or alkenyl each having up to 4 carbon atoms,
represent a group of the formula -NR²R³ or -OR⁶,
in which
R² and R³ are identical or different and denote hydrogen or straight-chain or branched alkyl having up to 4 carbon atoms,
R⁶ denotes straight-chain or branched alkyl or alkenyl having up to 4 carbon atoms, each of which is optionally substituted by cyclopropyl or phenyl,
or
A has one of the abovementioned meanings
and
B and D together denote a radical of the formula 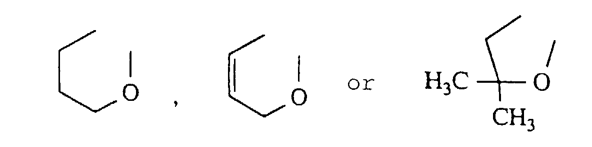
R¹ and R² are identical or different and
represent hydrogen or straight-chain or branched alkyl having up to 4 carbon atoms, or represent phenyl or benzyl, each of which is optionally substituted by fluorine, chlorine,
bromine, hydroxyl, trifluoromethyl or trifluoromethoxy or by straight-chain or branched alkyl or alkoxy each having up to 4 carbon atoms,
if appropriate in an isomeric form, and their salts.

4. Triazaspirodecanone-methylchromans according to Claim 1 for the treatment of diseases.

5. Process for the preparation of triazaspirodecanone-methylchromans of the general formula 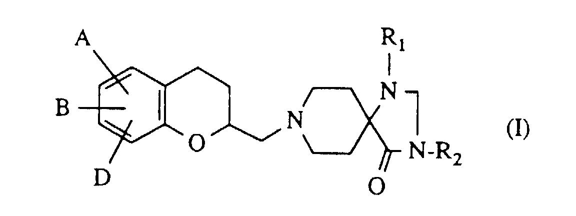 in which
A, B and D are identical or different and
represent hydrogen, halogen, cyano, azido, nitro, difluoromethyl, trifluoromethyl, difluoromethoxy, trifluoromethoxy, hydroxyl or carboxyl, or
represent straight-chain or branched alkyl, alkenyl, acyl or alkoxycarbonyl each having up to 8 carbon atoms, or
represent a group of the formula -NR³R⁴, -NR⁵-L-R⁶ or -OR⁷,
in which
R³, R⁴ and R⁵ are identical or different and denote hydrogen, straight-chain or branched alkyl having up to 8 carbon atoms, phenyl or benzyl,
L denotes the -CO- or -SO₂- group,
R⁶ denotes straight-chain or branched alkyl having up to 8 carbon atoms or benzyl, or denotes aryl having 6 to 10 carbon atoms, which is optionally substituted by halogen, hydroxyl, nitro, cyano, trifluoromethyl, trifluoromethoxy or by straight-chain or branched alkyl or alkoxy each having up to 6 carbon atoms,
R⁷ denotes straight-chain or branched alkyl or alkenyl each having up to 8 carbon atoms, each of which is optionally substituted by cycloalkyl having 3 to 6 carbon atoms or phenyl
or
A has one of the abovementioned meanings
and
B and D together form a 5- to 7-membered saturated, partially unsaturated or aromatic carbocyclic ring or heterocyclic ring having up to 2 heteroatoms from the series comprising S, N and O, where these rings can optionally have up to 2 carbonyl functions in the ring and are optionally monosubstituted or disubstituted by identical or different substituents from the series comprising straight-chain or branched alkyl or alkoxy each having up to 6 carbon atoms, hydroxyl, cycloalkyl having 3 to 6 carbon atoms, phenyl, halogen, cyano, nitro or in spiro fashion by a radical of the formula 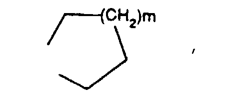
in which
m denotes a number 1 or 2,
and
R¹ and R² are identical or different and
represent hydrogen or straight-chain or branched alkyl, or
represent phenyl or benzyl, each of which is optionally monosubstituted to trisubstituted by identical or different substituents from the series comprising halogen, hydroxyl, cyano, difluoromethyl, difluoromethoxy, trifluoromethyl and trifluoromethoxy or by straight-chain or branched alkyl or alkoxy each having up to 8 carbon atoms,
if appropriate in an isomeric form, and their salts,
characterised in that compounds of the general formula (II) 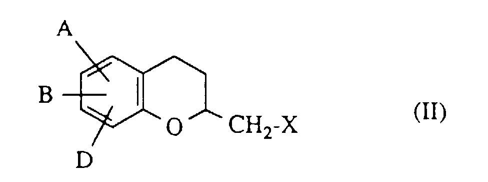 in which
A, B and D have the abovementioned meaning
and
X represents hydroxyl or a typical leaving group, such as tosyloxy, mesyloxy, chlorine or bromine,
are reacted in inert solvents, in the presence of a base and if appropriate of a catalyst, with compounds of the general formula (III) 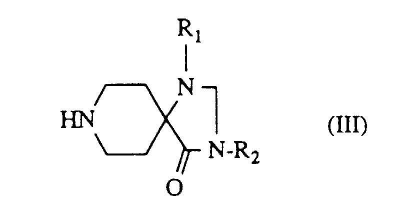 in which
R¹ and R² have the abovementioned meaning,
and if appropriate the substituents A, B and D are varied according to a customary method.

6. Process according to Claim 5, characterised in that it is carried out at a temperature of 0°C to +150°C.

7. Medicament containing at least one triazaspirodecan-one-methylchroman according to Claim 1.

8. Medicament according to Claim 7 for the treatment of diseases which are characterised by disorders of the serotoninergic and dopaminergic system.

9. Use of triazaspirodecanone-methylchromans according to Claim 1 for the production of medicaments.

10. Process for the production of medicaments according to Claim 7, characterised in that the triazaspirodecanone-methylchromans are converted into a suitable administration form, if appropriate with the aid of customary auxiliaries and excipients.

## Revendications

1. Triazaspirodécanone-méthylchromanes de formule générale 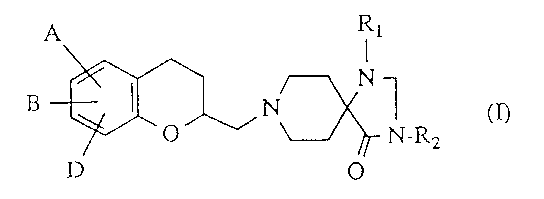 dans laquelle
A, B, et D sont identiques ou différents et représentent
de l'hydrogène, un halogène, un groupe cyano, azido, nitro, difluorométhyle, trifluorométhyle, difluorométhoxy, trifluorométhoxy, hydroxy ou carboxy, ou bien
un groupe alkyle, alcényle, acyle ou alkoxycarbonyle linéaire ou ramifié ayant chacun jusqu'à 8 atomes de carbone, ou un groupe de formule -NR³R⁴, -NR⁵-L-R⁶ ou -OR⁷,
dans laquelle
R³, R⁴ et R⁵ sont identiques ou différents et représentent de l'hydrogène, un groupe alkyle linéaire ou ramifié ayant jusqu'à 8 atomes de carbone, un groupe phényle ou benzyle,
L représente le groupe -CO- ou -SO₂-,
R⁶ est un groupe alkyle linéaire ou ramifié ayant jusqu'à 8 atomes de carbone ou un groupe benzyle, ou bien
un groupe aryle ayant 6 à 10 atomes de carbone, qui est substitué, le cas échéant, par un halogène, un radical hydroxy, nitro, cyano, trifluorométhyle, trifluorométhoxy ou par un radical alkyle ou alkoxy linéaire ou ramifié ayant chacun jusqu'à 6 atomes de carbone,
R⁷ est un groupe alkyle ou un groupe alcényle linéaire ou ramifié ayant chacun jusqu'à 8 atomes de carbone, qui sont substitués, le cas échéant, par un groupe cycloalkyle ayant 3 à 6 atomes de carbone ou par un groupe phényle,
ou bien
A a l'une des définitions indiquées ci-dessus
et
B et D forment conjointement un carbocycle ou un hétérocycle pentagonal à heptagonal, saturé, partiellement insaturé ou aromatique ayant jusqu'à 2 hétéroatomes de la série S, N ou O, qui peuvent porter éventuellement jusqu'à 2 fonctions carbonyle dans le noyau et qui sont substitués, le cas échéant, jusqu'à deux fois identiques ou différentes par un radical alkyle ou un radical alkoxy linéaire ou ramifié ayant chacun jusqu'à 6 atomes de carbone, un radical hydroxy, cycloalkyle ayant 3 à 6 atomes de carbone, phényle, halogéno, cyano, nitro, ou qui ont une substitution du type spiro par un reste de formule 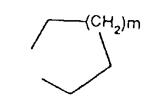
dans laquelle
m représente le nombre 1 ou 2,
et
R¹ et R² sont identiques ou différents et représentent de l'hydrogène ou un groupe alkyle linéaire ou ramifié, ou bien
un groupe phényle ou un groupe benzyle, qui sont substitués, le cas échéant, jusqu'à 3 fois identiques ou différentes par un radical halogéno, hydroxy, cyano, difluorométhyle, difluorométhoxy, trifluorométhyle, trifluorométhoxy ou
par un radical alkyle ou alkoxy linéaire ou ramifié ayant chacun jusqu'à 8 atomes de carbone,
le cas échéant sous une forme isomère, et leurs sels.

2. Triazaspirodécanone-méthylchromanes suivant la revendication 1,
dans lesquels
A, B, et D sont identiques ou différents et représentent de l'hydrogène, du fluor, du chlore, du brome, un groupe cyano, trifluorométhyle, difluorométhoxy, trifluorométhoxy ou hydroxy, ou bien un groupe alkyle, alcényle, acyle ou alkoxycarbonyle linéaire ou ramifié ayant chacun jusqu'à 6 atomes de carbone, ou un groupe de formule -NR³R⁴, -NR⁵-L-R⁶⁵ ou -OR⁷,
dans laquelle
R³, R⁴ et R⁵ sont identiques ou différents et représentent de l'hydrogène ou un groupe alkyle linéaire ou ramifié ayant jusqu'à 6 atomes de carbone,
L est le groupe -CO- ou -SO₂-
R⁶ représente un groupe alkyle linéaire ou ramifié ayant jusqu'à 6 atomes de carbone ou un groupe benzyle, ou bien
un groupe phényle, qui est substitué, le cas échéant, par du fluor, du chlore, du brome, un radical trifluorométhyle, trifluorométhoxy, hydroxy ou par un radical alkyle ou un radical alkoxy linéaire ou ramifié ayant chacun jusqu'à 4 atomes de carbone,
R⁷ est un groupe alkyle ou un groupe alcényle linéaire ou ramifié ayant chacun jusqu'à 6 atomes de carbone, qui sont substitués, le cas échéant, par un radical cyclopropyle, cyclopentyle, cyclohexyle ou phényle,
ou bien
A a l'une cdes définitions indiquées ci-dessus
et
B et D représentent conjointement un reste de formule 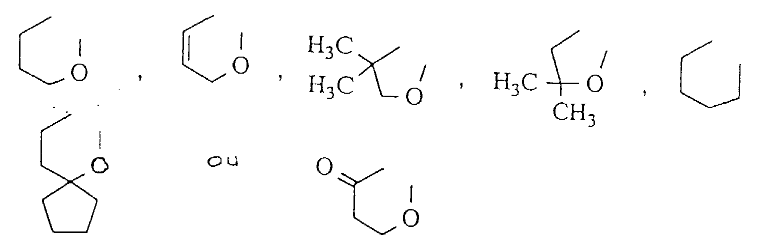
R¹ et R² sont identiques ou différents et représentent de l'hydrogène ou un groupe alkyle linéaire ou ramifié ayant jusqu'à 6 atomes de carbone, ou un groupée phényle ou un groupe benzyle qui sont substitués, le cas échéant, jusqu'à 2 fois identiques ou différentes par du fluor, du chlore, du brome, un radical hydroxy, cyano, trifluorométhyle, trifluorométhoxy ou par un radical alkyle ou un radical alkoxy linéaire ou ramifié ayant chacun jusqu'à 6 atomes de carbone,
le cas échéant sous une forme isomère, et leurs sels.

3. Triazaspirodécanone-méthylchromanes suivant la revendication 1 dans lesquels
A, B, et D sont identiques ou différents et représentent de l'hydrogène, du fluor, du chlore, du brome, un groupe cyano, trifluorométhyle, trifluorométhoxy ou hydroxy,
un groupe alkyle ou un groupe alkoxy linéaire ou ramifié ayant chacun jusqu'à 4 atomes de carbone,
un groupe de formule -NR²R³ ou -OR⁶,
dans laquelle
R² et R³ sont identiques ou différents et représentent de l'hydrogène ou un groupe alkyle linéaire ou ramifié ayant jusqu'à 4 atomes de carbone,
R⁶ est un groupe alkyle ou un groupe alcényle linéaire ou ramifié ayant jusqu'à 4 atomes de carbone, qui sont substitués, le cas échéant, par un radical cyclopropyle ou phényle,
ou bien
A a l'une des définitions indiquées ci-dessus
et
B et D forment ensemble un reste de formule 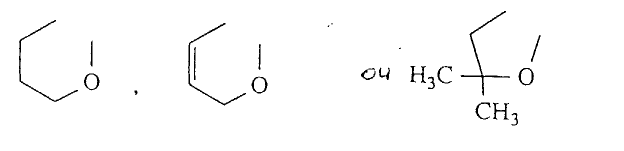
R¹ et R² sont identiques ou différents et représentent de l'hydrogène ou un groupe alkyle linéaire ou ramifié ayant jusqu'à 4 atomes de carbone, ou bien
un groupe phényle ou un groupe benzyle qui sont substitués, le cas échéant, par du fluor, du chlore, du brome, un radical hydroxy, trifluorométhyle, trifluorométhoxy ou par un radical alkyle ou un radical alkoxy linéaire ou ramifié ayant chacun jusqu'à 4 atomes de carbone,
le cas échéant sous une forme isomère, et leurs sels.

4. Triazaspirodécanone-méthylchromanes suivant la revendication 1, destinés au traitement de maladies.

5. Procédé de production de triazaspirodécanone-méthylchromanes de formule générale 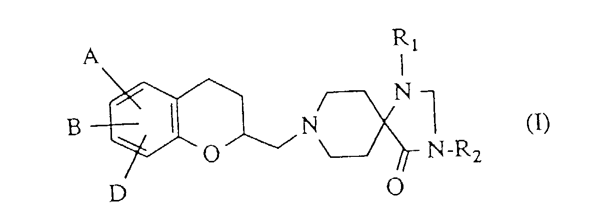 dans laquelle
A, B, et D sont identiques ou différents et représentent
de l'hydrogène, un halogène, un groupe cyano, azido, nitro, difluorométhyle, trifluorométhyle, difluorométhoxy, trifluorométhoxy, hydroxy ou carboxy, ou bien
un groupe alkyle, alcényle, acyle ou alkoxycarbonyle linéaire ou ramifié ayant chacun jusqu'à 8 atomes de carbone, ou un groupe de formule -NR³R⁴, -NR⁵-L-R⁶ ou -OR⁷,
dans laquelle
R³, R⁴ et R⁵ sont identiques ou différents et représentent de l'hydrogène, un groupe alkyle linéaire ou ramifié ayant jusqu'à 8 atomes de carbone, un groupe phényle ou benzyle,
L représente le groupe -CO- ou -SO₂-,
R⁶ est un groupe alkyle linéaire ou ramifié ayant jusqu'à 8 atomes de carbone ou un groupe benzyle, ou bien
un groupe aryle ayant 6 à 10 atomes de carbone, qui est substitué, le cas échéant, par un halogène, un radical hydroxy, nitro, cyano, trifluorométhyle, trifluorométhoxy ou par un radical alkyle ou alkoxy linéaire ou ramifié ayant chacun jusqu'à 6 atomes de carbone,
R⁷ est un groupe alkyle ou un groupe alcényle linéaire ou ramifié ayant chacun jusqu'à 8 atomes de carbone, qui sont substitués, le cas échéant, par un groupe cycloalkyle ayant 3 à 6 atomes de carbone ou par un groupe phényle,
ou bien
A a l'une des définitions indiquées ci-dessus
et
B et D forment conjointement un carbocycle ou un hétérocycle pentagonal à heptagonal, saturé, partiellement insaturé ou aromatique ayant jusqu'à 2 hétéroatomes de la série S, N ou O, qui peuvent porter éventuellement jusqu'à 2 fonctions carbonyle dans le noyau et qui sont substitués, le cas échéant, jusqu'à deux fois identiques ou différentes par un radical alkyle ou un radical alkoxy linéaire ou ramifié ayant chacun jusqu'à 6 atomes de carbone, un radical hydroxy, cycloalkyle ayant 3 à 6 atomes de carbone, phényle, halogéno, cyano, nitro, ou qui ont une substitution du type spiro par un reste de formule 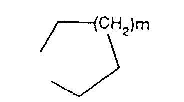
dans laquelle
m représente le nombre 1 ou 2,
et
R¹ et R² sont identiques ou différents et représentent
de l'hydrogène ou un groupe alkyle linéaire ou ramifié, ou bien
un groupe phényle ou un groupe benzyle, qui sont substitués, le cas échéant, jusqu'à 3 fois identiques ou différentes par un radical halogéno, hydroxy, cyano, difluorométhyle, difluorométhoxy, trifluorométhyle, trifluorométhoxy ou par un radical alkyle ou alkoxy linéaire ou ramifié ayant chacun jusqu'à 8 atomes de carbone,
le cas échéant sous une forme isomère, et de leurs sels,
caractérisé en ce qu'on fait réagir des composés de formule générale (II) 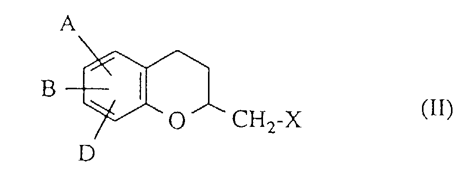 dans laquelle
A, B et D ont la définition indiquée ci-dessus
et
X est un groupe hydroxy ou un groupe partant caractéristique tel que tosyloxy, mésyloxy, chloro ou bromo,
dans des solvants inertes, en présence d'une base et, le cas échéant, d'un catalyseur, avec des composés de formule générale (III) 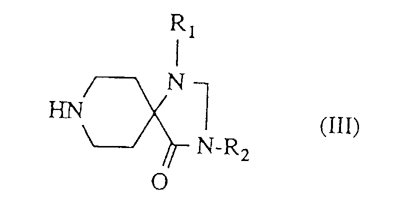 dans laquelle
R¹ et R² ont la définition indiquée ci-dessus,
et, le cas échéant, on fait varier les substituants A, B et D selon une méthode classique.

6. Procédé suivant la revendication 5, caractérisé en ce qu'on opère à une température de 0°C à +150°C.

7. Médicament contenant au moins un triazaspirodécanone-méthylchromane suivant la revendication 1.

8. Médicament suivant la revendication 7, destiné au traitement de maladies qui sont caractérisées par des troubles du système sérotoninergique et dopaminergique.

9. Utilisation de triazaspirodécanone-méthylchromanes suivant la revendication 1 pour la préparation de médicaments.

10. Procédé de préparation de médicaments suivant la revendication 7, caractérisé en ce qu'on fait prendre une forme d'administration appropriée à des triazaspirodécanone-méthylchromanes, le cas échéant, à l'aide de substances auxiliaires et de supports classiques.
